# EUROPEAN PATENT APPLICATION

(11) **EP 1 992 334 A1**
(43) Date of publication of application: **19.11.2008**
(21) Application number: 07107708.5
(22) Date of filing: 08.05.2007
(51) Int. Cl.: A61K 9/08, A61K 31/167, A61K 47/26, A61K 9/00

(54) **Storage-stable formulation of oxidation-sensitive phenolic drug, especially paracetamol, comprises aqueous drug solution deoxygenated by a temperature-controlled manufacturing process of the formulation**

(71) Applicant: Docpharma NV/SA, 3001 Heverlee (BE)
(72) Inventor: Al-Dandachi Atassi, Khaled, 1190 Brussel (BE)
(74) Representative: Brants, Johan P.E.

(57) **Abstract**

The present invention relates to a liquid formulation, stable to oxidation, based on a phenolic active principle susceptible to oxidation such as paracetamol in an aqueous solvent and to a method for preparing such formulation. The formulation and the method are characterized in that the active principle is admixed in the aqueous solvent having a temperature between 65°C and 95°C, a pH between 5.0 and 6.0 and an oxygen concentration below 2 ppm.

## Description

The object of the present invention is a new formulation and a new method for producing injectable aqueous solutions with active principles, in particular active principles that are useful in therapeutics and susceptible to oxygen, and a procedure for preparation of these methods of packaging, and their utilization.

Paracetamol (INN of acetaminophen or N-(4-hydroxy-phenyl)acetamide) is an analgesic and an antipyretic widely used in hospitals. It is desirable to have available stable liquid pharmaceutical formulations of this active principle for administration by injection, in particular for intravenous infusion.

It is known that paracetamol in aqueous solution is liable to undergo hydrolysis to form p-aminophenol, which is itself liable to degrade into quinoneimine (cf. for example J. E. Fairbrother, "Acetaminophen" in Analytical Profiles of Drug Substances, 1974, vol. 3, pp. 1-109). The rate of degradation of paracetamol increases with increasing temperature and light. This rate is minimal at a pH in the region of 6 (K. T. Koshy et al., 1961, J. Pharm. Sci. 50, pp. 116-118).

It is known practice to add a buffer and an antioxidant or free-radical scavenger to stabilize paracetamol in solution.

WO 02/072 080, for example, describes stable aqueous paracetamol solutions for infusion comprising a buffer of pH 5.5 to 6.5 and an antioxidant chosen from ascorbic acid and a derivative bearing a thiol function such as cysteine or acetyl-cysteine.

EP 0 916 347 discloses-paracetamol solutions based on a mixture of water and of alcoholic solvents comprising a buffer of pH 5.5 to 5.6 and metabisulfite as antioxidant.

EP 0 859 329 describes a deoxygenation process by which the aqueous solvent is deoxygenated by bubbling into an inert gas, such as nitrogen.

The prior-art stabilized injectable solutions of paracetamol have the drawback of causing a potential irritant, allergenic and/or carcinogenic effect in certain patients, on account of the toxicity of the antioxidant they contain. Furthermore, their stability requires the removal of the oxygen and other oxidizing agents from the aqueous medium or by the use of a water-soluble inert gas. These methods have however the disadvantage of requiring a considerable amount of time. The bubbling of nitrogen is the method the most practiced within the pharmaceuticals industry. Another disadvantage is that the oxidation products lead to the formation of colored compounds, thus making the aqueous solution unsuitable for therapeutic applications. All the above methods have a certain efficacy. However, oxygen shows a very great facility to dissolve in water, making it necessary to ensure that the solution, once deoxygenated, does not subsequently come into contact with atmospheric air.

The main object of the present invention is to provide a formulation and a method for aqueous formulations with active principles susceptible to oxidation, which can notably be utilized in injectable preparations being stable over a long period.

Therefore, the invention provides in a first aspect liquid formulations that are stable to oxidation and that are based on a phenolic active principle susceptible to oxidation such as paracetamol in an aqueous solvent. The formulations are characterized in that the active principle is admixed in the aqueous solvent having a temperature between 65°C and 95°C, a pH between 5.0 and 6.0 and an oxygen concentration below 2 ppm.

In an embodiment, the invention relates to a formulation as defined herein, wherein the aqueous solvent has a temperature between 70°C and 90° or between 75°C and 85°C.

In another embodiment, the invention relates to a formulation as defined herein, wherein the aqueous solvent has a pH between 5.6 and 5.7 and preferably around 5.5.

In another embodiment, the invention relates to a formulation as defined herein, wherein the aqueous solvent has an oxygen content lower than 2 ppm, lower than 1.5 ppm or lower than 1 ppm.

In yet another embodiment, the invention relates to a formulation as defined herein, wherein the aqueous solvent comprises water, an isotonizing agent and a buffer agent.

In yet another embodiment, the invention relates to a formulation as defined herein, wherein the mass ratio of the isotonizing agent/active principle, for instance mannitol/paracetamol is between 5/1 and 3/1.

The invention further provides a formulation as defined herein wherein the obtained mixture or the obtained formulation of solvent/ active principle, for instance solvent/paracetamol, is filtered at a temperature between 65°C and 95°C, and for instance at a temperature between 70°C and 90°C or between 75°C and 85°C.

In second aspect the invention relates to a method for the production of a formulation as defined herein, comprising the steps of:
- dissolving a phenolic active principle susceptible to oxidation such as paracetamol in an aqueous solvent having a temperature between 65°C and 95°C, and having pH between 5.0 and 6.0 in a reaction vessel,
- closing of the reaction vessel and replacing the remaining air in the vessel by an inert gas, such as nitrogen, and
- filtering the obtained formulation (mixture) at a temperature between 65°C and 95°C, whereby the oxygen content in the obtained formulation is below 2 ppm during the complete method.

Here and in the following text, the term *"phenolic active principle susceptible to oxidation"* means any substance, which may or may not be medicinal, comprising a phenolic structure and/or functions supported by the phenolic structure which react easily with oxygen, and which degrades forming oxidation products, coloured or colourless, or hydrolysis products or polymerization products.

Amongst the easily oxidizable active principles that can be incorporated into the aqueous solutions of the invention, the following may be cited more particularly: phenols or aminophenols, such as paracetamol, epinephrine, norepinephrine, adrenalone, isoprenaline, orciprenaline, isoxuprine, phenylephrine or dobutamine; the following may be cited as aromatic amines: procaine, bupivacaine, tetracaine, butoform, L-dopa or Carbidopa; the following one may be cited as aminoketones: Propaphenone; the following ones may be cited as aminoglucosides: the gentamycines, amikacine, dibekacine, netilmycin, sisomycin, tobramycin, micronomycine; as phenothiazines, promethazine; as hydroaromatic molecules, riboflavin, 9-amino dihydro acridine; further cortisonic derivatives may be cited, such as dexamethasone, betamethasone, triamcinolone, fluocinonide, flunisolide, fluocinolone acetonide, fluocortolone, Clobetasone and their derivatives, beclometasone and its esters; Tetracycline derivatives, such as Doxycycline or Minocycline.

For the purpose of improving the stability of such medicinal active principles which are susceptible to oxidation, and thus to overcome the disadvantages described above, the present invention provides a method and a formulation wherein the oxygen is eliminated during the preparation of the formulation and during the subsequent filtration of the obtained mixture by a temperature-controlled manufacturing wherein the temperature is initially set at and maintained within 65°C and 95°C. The invention therefore provides in a first aspect a liquid, stable to oxidation formulation based on an active principle of a phenolic nature susceptible to oxidation, while being able to be preserved for a prolonged period, such as paracetamol in an aqueous solvent, characterized in that the active ingredient is admixed in the aqueous solvent having a temperature between 65°C and 95°C, a pH between 5.0 and 6.0 and an oxygen concentration below 2 ppm.

Within the framework of the industrial manufacture of injectable solutions, it has made been possible by the present invention to eliminate the oxygen content in the bulk solutions in airtight tanks by maintaining the temperature between 65°C and 95°C, and to keep the prepared solution away from the air. The tanks are therefore preferably being fluxed with an inert gas to replace the air by said inert gas. Within this industrial manufacturing process the obtained solution has to be filtered in a filtration unit. As the present invention is directed towards a temperature-controlled manufacturing process the subsequent filtration of the obtained mixture also preferably takes place between 65°C and 95°C. Precautions that may be taken for this purpose, especially with the filtration procedure is to replace the air in the filtration unit, or by an inert gas or by a fraction of the obtained mixture, which will be evacuated afterwards and prior to the actual filtration. Eventually, the filling and packaging of the bottles can also take place with the addition of an inert gas, such as nitrogen.

Although the invention is not limited to paracetamol only, for reasons of clarity of the present application, paracetamol formulations according to the invention will be exemplified in further details.

Preferably, the formulations of the present invention are prepared and filtered at a temperature between 65°C and 95°C, and for instance between 70°C and 90°C or between 75°C and 85°C, at preferably of at least 70°C, and a pH between 5.0 and 6.0 and preferably between 5.6 and 5.7, and preferably around 5.5.

Preferred formulations contain a phenolic active principle susceptible to oxidation such as paracetamol, a buffer and an isotonic (isotonizing) agent.

A formulation according to the present invention contains a buffer with a pKa of between 4.5 and 6.5 and preferably between 5.0 and 6.2. This buffer will advantageously be chosen from citrate buffer, phosphate buffer, phosphate-citrate buffer, bicarbonate buffer, tartrate buffer and acetate buffer, preferably from citrate buffer, phosphate buffer and phosphate-citrate buffer, or a mixture of these buffers. Most preferably, the buffer is disodium phosphate dihydrate (Na₂HPO₄2H₂O).

The present formulations for injection further contain an isotonic agent, intended to create an osmotic pressure in the region of that of physiological saline. The isotonizing agent also referred to as isotonic agent herein may be a polyol, a sugar, a linear or cyclic glucitol having from 2 to 10 carbon atoms selected from mannitol, sorbitol, inositol, glucose and glycerol. This isotonic agent can be chosen from sodium chloride and glucose. A preferred isotonic agent is mannitol.

The formulation of the invention is generally prepared as follows. First an aqueous solvent or solution is prepared by mixing together water suitable for injection (WFI), a buffer and an isotonic agent, at a pH from 5 to 6 and preferably at a pH of about 5.5. The pH can be adjusted by q.s. of NaOH or HCl. Optionally one or more other water-miscible solvent(s), and/or surfactants might be present. Then, paracetamol is admixed to the aqueous solvent, the solvent being provided at a temperature of between 65°C and 95°C. Mixing is followed by the filtration of the obtained formulation. The temperature of the complete process is maintained during admixing and during the filtration between 65°C and 95°C, preferably between 75°C and 85°C and most preferably around 80°C.

The invention also relates to a formulation as defined above that may be obtained via this process.

An important advantage of the present process comprises admixing of the active principle to the aqueous solvent that has a temperature of between 65°C and 95°C. Advantageously, the aqueous solvent is not cooled prior to admixture of the active principle, which constitutes not only a gain in the preparation time of the formulation (no need to use heat exchangers to cool the aqueous solvent), but also permits to obtain a suitable oxygen concentration. Further, also filtration of the obtained formulation advantageously takes place at a temperature between 65°C and 95°C without cooling of the solution.

The invention is described in greater detail in the examples below, which are given as nonlimiting illustrations. In these examples, the temperature is room temperature or is expressed in degrees Celsius, and the pressure is atmospheric pressure. The water and all the reagents used are of injectable grade.

Moreover, all the examples form an integral part of the invention, as does any characteristic of the description including the examples, which appears to be novel with respect to any prior art, in the form of a general characteristic rather than of a particular characteristic of the example.

### EXAMPLE 1

### PREPARATION OF LIQUID PHARMACEUTICAL FORMULATIONS ACCORDING TO THE INVENTION

Formulations were prepared by admixing paracetamol to a solution of water for injection, buffer (phosphate buffer) and isotonic agent (mannitol), filtration and filling of glass vials or bottles. Up to the filling of the glass bottles or vials the process was maintained at temperatures between 65°C and 95°C, also during filtration. These bottles can then be sterilized for 15 minutes at 121°C.

### Formulation 1

| **Component** | **Quantity** |
|---|---|
| Paracetamol | 1000 mg |
| Mannitol (isotonizing agent) | 3909.6 mg |
| Di sodium Phosphate dehydrate (buffer) | 15 mg |
| HCl 0.1 N or NaOH 0.1 N | q.s. pH = 5.5 |
| WFI | 100 ml |
| Nitrogen | q.s. |

The process of formulation and filtration of paracetamol solution is performed at a temperature between 65°C and 95°C, and for instance between 75 °C and 85 °C in order to avoid oxygen contamination.

The required tubes and filters are usually sterilized, so this material can be used at the above-mentioned temperature. All manufacturing steps are performed quickly and without any unnecessary interruption in order to avoid contact of the solution with air and to keep the solution at the required temperature between 65°C and 95°C and for instance between 75 °C and 85 °C.

In a first step a reaction vessel equipped with a stirrer is provided with about 90% of the total required quantity WFI (water for injection), which under some circumstances and preferably can be taken directly from a WFI loop at temperature between 75°C and 85°C. The weights are registered. Then the following steps are performed: add smoothly and without stirring the required amount of isotonic agent, preferably mannitol and the buffer, preferably di sodium phosphate dehydrate. Close the vessel and put it under 0.22 µm filtered nitrogen pressure. Stir the obtained mixture until complete dissolution (normally about 1 to 2 minutes). Reopen the vessel and measure and adjust the pH. Provide therefore within the vessel a special electrode for pH measurement at temperature between 80 °C and 100 °C and under minimum speed stirring, adjust the pH to 5.5 with e.g. HCl or NaOH 0.1N. Once the pH has been set at about 5.5, and preferably at 5.5, stop stirring the solution and add the required amount of paracetamol. Close the vessel and put it under (0.22 µm filtered) nitrogen pressure. Stir the obtained mixture until complete dissolution of paracetamol (about 1 minute) and reopen the vessel and bring quickly to final volume with WFI between 75 °C and 85 °C taking into consideration the density thereof. Close the vessel and put it under 0.22 µm filtered nitrogen pressure. Stir about 1 minute. Open the vessel and check the pH. Adjust at pH 5.5 with HCL 0.1 N or NaOH 0.1 N if necessary. Close the vessel and put it under (0.22 µm filtered) nitrogen pressure.

In a second step, the filtration of the solution takes place at a temperature between 65°C and 95°C and for instance between 75 °C and 85 °C without cooling of the solution. A 0.22 µm filter with sanitary flange inlet and outlet connections and integral vent and drain valves for immediate installation can be used. The filtration vessel is certified for pressure and equipped with 0.22 µm vent filter and 0.22 µm nitrogen filter. Replace the air inside the filtration vessel by 0.22 µm filtered nitrogen and keep it under nitrogen pressure.

Connect the tube IN to the inlet flange of the filter and connect the other side of the tube to the compounding vessel. Connect the tube OUT to the outlet flange of the filter. Apply nitrogen pressure on the solution in the compounding vessel and discard about 300 ml of the solution by the tube that is connected to the outlet flange of the filter. Purge the filter by the drain valve and repeat this operation until no bubbles are present. Connect the tube OUT to the outlet flange of the filter to the filtration vessel. Apply 0.22 µm filtered nitrogen pressure on the solution in the compounding vessel to push the solution throughout the filter and open the valve of the vent filter of the filtration vessel. Achieve the filtration and stop the filtration when about 1 litre of solution is still remaining in the compounding vessel. Close off the valve of the vent filter of the filtration vessels and put it under (0.22 µm filtered) nitrogen pressure. Keep the solution in the filtration vessel until the temperature is about 25-27 °C or at room temperature. In this case, the vessel is kept until the next day. If the filtration vessel is equipped with a jacket, cool the solution and continue the operations. The special purging step in the process is preferred to minimize the risk of oxygenation of the mixture.

During the above-mentioned steps the temperature preferably remained at at least 75°C and the oxygen concentration was lower than 2 ppm.

The filling of the solution was performed using known techniques by replacing the air in vials by (0.22 µm filtered) nitrogen until the nitrogen goes out of the needles of the foiling machine. Fill the solution under nitrogen flushing before and after filling.

Finally, the filled vials can be sterilized at 121 °C during 15 minutes.

## Claims

1. Liquid, stable to oxidation formulation, based on a phenolic active principle susceptible to oxidation such as paracetamol in an aqueous solvent, **characterized in that** the active principle is admixed in the aqueous solvent having a temperature between 65°C and 95°C, a pH between 5.0 and 6.0 and an oxygen concentration below 2 ppm.

2. Formulation according to claim 1, wherein the aqueous solvent has a temperature between 75°C and 85°C.

3. Formulation according to claim 1 or 2, wherein the aqueous solvent has a pH between 5.6 and 5.7, and preferably around 5.5.

4. Formulation according to any of the claims 1 to 3, wherein the aqueous solvent comprises water, an isotonizing agent and a buffer agent.

5. Formulation according to claim 4, wherein the isotonizing agent is a polyol, a sugar, a linear or cyclic glucitol having from 2 to 10 carbon atoms selected from mannitol, sorbitol, inositol, glucose and glycerol, and preferably mannitol.

6. Formulation according to claim 5 wherein the mass ratio of mannitol/paracetamol is between 5/1 and 3/1.

7. Formulation according to any of claims 1 to 6, wherein the obtained mixture solvent/paracetamol is filtered at a temperature between 65°C and 95°C.

8. Method for the production of a formulation according to any of the previous claims 1 to 7, comprising the steps of:
- dissolving paracetamol in an aqueous solvent having a temperature between 65°C and 95°C, and having a pH between 5.0 and 6.0 in a reaction vessel,
- closing of the reaction vessel and replacing the remaining air in the vessel by an inert gas, such as nitrogen, and
- filtering the obtained mixture at a temperature between 65°C and 95°C, such that the oxygen content in the obtained formulation is below 2 ppm during the complete method.
